(19) Europäisches Patentamt
European Patent Office
Office européen des brevets

(11) **EP 3 416 542 B1**

(12) **EUROPEAN PATENT SPECIFICATION**

(45) Date of publication and mention
of the grant of the patent:
**28.07.2021 Bulletin 2021/30**

(51) Int Cl.:
*A61B 5/02* (2006.01)     *A61B 5/00* (2006.01)
*A61B 5/08* (2006.01)     *A61B 5/145* (2006.01)
*A61B 5/20* (2006.01)     *A61B 5/107* (2006.01)

(21) Application number: **17710394.2**

(22) Date of filing: **04.02.2017**

(86) International application number:
**PCT/IB2017/050620**

(87) International publication number:
**WO 2017/141131 (24.08.2017 Gazette 2017/34)**

(54) **SYSTEM AND METHOD FOR DETERMINING A HEMODYNAMIC INSTABILITY RISK SCORE FOR PEDIATRIC SUBJECTS**

SYSTEM UND VERFAHREN ZUR BESTIMMUNG EINES HÄMODYNAMISCHEN INSTABILITÄTSRISIKOS FÜR PÄDIATRISCHE SUBJEKTE

SYSTÈME ET PROCÉDÉ DE DÉTERMINATION D'UN SCORE DE RISQUE D'INSTABILITÉ HÉMODYNAMIQUE POUR SUJETS PÉDIATRIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.02.2016 US 201662297208 P**

(43) Date of publication of application:
**26.12.2018 Bulletin 2018/52**

(73) Proprietor: **Koninklijke Philips N.V.**
**5656 AG Eindhoven (NL)**

(72) Inventors:
• **POTES BLANDON, Cristhian Mauricio**
**5656 AE Eindhoven (NL)**
• **XU, Minnan**
**5656 AE Eindhoven (NL)**
• **CONROY, Bryan**
**5656 AE Eindhoven (NL)**

(74) Representative: **Philips Intellectual Property & Standards**
**High Tech Campus 5**
**5656 AE Eindhoven (NL)**

(56) References cited:
**US-A1- 2014 073 973     US-A1- 2014 323 846**
**US-A1- 2015 145 691**

• **HANQING CAO ET AL: "Predicting ICU hemodynamic instability using continuous multiparameter trends", ENGINEERING IN MEDICINE AND BIOLOGY SOCIETY, 2008. EMBS 2008. 30TH ANNUAL INTERNATIONAL CONFERENCE OF THE IEEE, IEEE, PISCATAWAY, NJ, USA, 20 August 2008 (2008-08-20), pages 3803-3806, XP031508837, ISBN: 978-1-4244-1814-5**

**Description**

TECHNICAL FIELD

The present disclosure pertains to a system and method for determining a hemodynamic instability risk score for a pediatric subject.

BACKGROUND

Hemodynamic instability (shock) is a major cause of morbidity and mortality in the pediatric population. Shock results from inadequate oxygen delivery to meet metabolic demands, leading to organ dysfunction and death if it is not treated early. Early recognition of shock and aggressive therapy has been shown to decrease mortality in children. However, normal vital signs and laboratory values for healthy children have limited application when detecting clinical deterioration and hemodynamic instability. This is because many clinical features such as blood pressure, heart rate, and respiratory rate normally vary as children grow and age such that any baseline value used to detect deterioration is often not accurate.

US 2014/323846 describes a system for determining a hemodynamic status of an individual through a blood pressure related index. US 2015/145691 describes a method of rendering hemodynamic instability index indicator information.

SUMMARY

Accordingly, one or more aspects of the present disclosure relate to a system configured to determine a hemodynamic instability risk score for a pediatric subject, according to the independent claim. The system comprises one or more hardware processors. The one or more hardware processors are configured by machine-readable instructions to obtain an age of the subject; obtain feature values for a plurality of features associated with physiological characteristics of the subject; inputting the age of the subject and each feature value into a respective bivariate classifier that is configured to output a respective feature contribution prediction score for each feature, wherein each bivariate classifier determines one or more feature value thresholds for the respective feature that indicates risk of hemodynamic instability in the subject. The feature value thresholds are determined based on the age of the subject, such that the one or more feature value thresholds for individual features including heart rate, blood pressure, and shock index are quadratic functions of the age of the subject. The one or more hardware processors are further configured to use each bivariate classifier to determine feature contribution prediction score for the respective feature based on whether the obtained feature value breaches the one or more of the determined feature value thresholds for the individual features; and aggregate the feature contribution prediction scores to determine the hemodynamic instability risk score for the subject.

Another aspect of the present disclosure relates to a method according to the independent claim for determining a hemodynamic instability risk score for a pediatric subject with a determination system. The system comprises one or more hardware processors configured to execute machine-readable instructions. The method comprises obtaining an age of the subject; obtaining feature values for a plurality of features associated with physiological characteristics of the subject; inputting the age of the subject and each feature value into a respective bivariate classifier that outputs a respective feature contribution prediction score for each feature, and wherein each bivariate classifier determines one or more feature value thresholds for the respective feature that indicates risk of hemodynamic instability in the subject. The feature value thresholds are determined based on the age of the subject, wherein the one or more feature value thresholds for individual features including heart rate, blood pressure, and shock index are quadratic functions of the age of the subject. The method further comprises using each bivariate classifier to determine a feature contribution prediction score for the respective feature based on whether the obtained feature value breaches the one or more of the determined feature value thresholds for the individual features; and aggregating the feature contribution prediction scores to determine the hemodynamic instability risk score for the subject.

These and other objects, features, and characteristics of the present disclosure, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the disclosure.

BRIEF DESCRIPTION OF THE DRAWINGS

FIG. 1 is a schematic illustration of a system configured to determine a hemodynamic instability risk score for a pediatric subject.
FIG. 2 illustrates determining a hemodynamic instability risk score.
FIG. 3 is a visual illustration of a bivariate classifier including two decision stumps for noninvasive shock index.
FIG. 4 illustrates a summary of the operations performed by the system.
FIG. 5 illustrates a method for determining a hemodynamic instability risk score for a pediatric patient.

DETAILED DESCRIPTION OF EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (i.e., a plurality).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

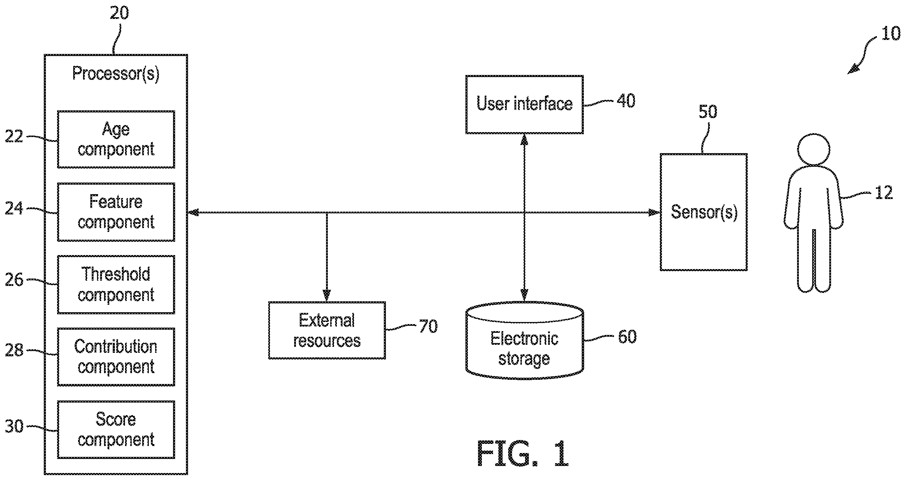

FIG. 1 is a schematic illustration of a system 10 configured to determine a hemodynamic instability risk score for a pediatric subject 12. System 10 comprises a hemodynamic instability indicator algorithm that helps pediatric intensive care unit clinicians and/or other caregivers (e.g., doctors, nurses, parents, etc.) focus care on subjects at increased risk of deterioration (e.g., inadequate oxygen delivery by the respiration system of subject 12 to meet metabolic demands, which may lead to organ dysfunction and/or death). System 10 receives vital signs along with laboratory data, patient demographic information, and/or other heath information related to subject 12 (feature values associated with physiological characteristics of subject 12), analyzes the received information based on the age of subject 12, and outputs a score that quantifies the risk of hemodynamic instability in subject 12.

Early detection of and/or diagnosis of hemodynamic instability (shock) in the pediatric population is very difficult and requires a high amount of clinical expertise. Diagnosis of shock typically requires a physical examination as well as a comprehensive evaluation and understanding of the feature values (e.g., vital signs, laboratory values) and/or other heath information related to subject 12. However, the physiology of children changes as they grow and age. Clinical features such as heart rate, respiration rate, blood pressure, and/or other features may change dramatically over the course of a few months to a year. For example, a normal heart rate for children less than one year old is between about 100-160 beats per minute. As a child ages, the normal heart rate decreases to about 60-100 beats per minute. System 10 automatically defines age adjusted thresholds for vital signs, laboratory values, and/or other health information that are used to determine the hemodynamic instability risk score. Caregivers do not currently have a clinical decision support tool that integrates this clinical information, considers the age of a subject, and provides an indicator of the risk of hemodynamic instability (shock) in pediatric subjects. In some embodiments, system 10 comprises one or more of a processor 20, a user interface 40, a sensor 50, electronic storage 60, external resources 70, and/or other components.

Processor(s) 20 are configured to provide information processing capabilities in system 10. As such, processor 20 may comprise one or more of a digital processor, an analog processor, a digital circuit designed to process information, an analog circuit designed to process information, a state machine, and/or other mechanisms for electronically processing information (including microprocessors, field-programmable gate arrays, application-specific integrated circuits, and other similar devices). Although processor 20 is shown in FIG. 1 as a single entity, this is for illustrative purposes only. In some embodiments, processor 20 may comprise a plurality of processing units. These processing units may be physically located within the same device (e.g., a server), or processor 20 may represent processing functionality of a plurality of devices operating in coordination (e.g., a server, computing devices associated with caregivers, computing devices associated with subject 12 and/or other users, sensors 50, user interface 40, devices that are part of external resources 70, and/or other devices.)

As shown in FIG. 1, processor 20 is configured via machine-readable instructions to execute one or more computer program components. In various embodiments, such instructions may be stored in a memory device such as L1/L2/L3 cache, system memory, or a storage device. As used herein, the term "non-transitory" will be understood to apply to both volatile memory (*e.g.*, SRAM and DRAM devices) and non-volatile memory (*e.g.*, flash, magnetic, and optical memory devices) but to exclude transitory signals. The one or more computer program components may comprise one or more of an age component 22, a feature component 24, a threshold component 26, a contribution component 28, a score component 30, and/or other components. Processor 20 may be configured to execute components 22, 24, 26, 28, and/or 30 by software; hardware; firmware; some combination of software, hardware, and/or firmware; and/or other mechanisms for configuring processing capabilities on processor 20.

It should be appreciated that although components 22, 24, 26, 28, and 30 are illustrated in FIG. 1 as being co-located within a single processing unit, in embodiments in which processor 20 comprises multiple processing units, one or more of components 22, 24, 26, 28, and/or 30 may be located remotely from the other components. The description of the functionality provided by the different components 22, 24, 26, 28, and/or 30 described below is for illustrative purposes, and is not intended to be limiting, as any of components 22, 24, 26, 28, and/or 30 may provide more or less functionality than is described. For example, one or more of components 22, 24, 26, 28, and/or 30 may be eliminated, and some or all of its functionality may be provided by other components 22, 24, 26, 28, and/or 30. As another example, processor 20 may be configured to execute one or more additional components that may perform some or all of the functionality attributed below to one of components 22, 24, 26, 28, and/or 30.

Age component 22 is configured to obtain the age of subject 12. In some embodiments, the age of subject 12 is measured in years, months, weeks, days, and/or other increments. In some embodiments, the obtained age of subject 12 is the age of subject 12 at a time when feature values (described below) for subject 12 are obtained. This may include the current age of subject 12 and/or a past age of subject 12 that corresponds to a time when the feature values were obtained. For example, for lab results obtained days and/or weeks prior to a current day, the obtained age for subject 12 may correspond to a date of the lab test. As another example, for a current heart rate measurement, the obtained age for subject 12 may be the current age of subject 12.

In some embodiments, age component 22 is configured to obtain the age of subject 12 by facilitating entry and/or selection of the age of subject 12 via user interface 40 (described below) and/or other interfaces. For example, age component 22 may be configured such that a caregiver of subject 12 enters the age of subject 12 via an age entry field of a graphical user interface presented to the caregiver via user interface 40. In some embodiments, the age of subject 12 is obtained by age component 22 from one or more external clinical databases that include medical information indicating the age of subject 12 and/or other information. The one or more external clinical databases may be included in, for example, electronic storage 60, external resources 70, and/or in other locations. In some embodiments, the medical information in the external clinical databases is collected by one or more of a caregiver in outpatient or inpatient settings, collected during previous visits to a doctor's office and/or the hospital, collected during a current doctor's office and/or hospital visit, and/or collected at other times. In some embodiments, age component 22 is configured such that obtaining the age of subject 12 includes determining the age of subject 12 based on information entered and/or selected via user interface 40 and/or information obtained from an external database. For example, age component 22 may obtain the birth date of subject 12, compare the birth date to a current date, and then determine the age of subject 12 based on this comparison.

Feature component 24 is configured to obtain feature values for one or more features associated with physiological characteristics of subject 12. The one or more features include invasive shock index (bpm/mmHg), mean airway pressure ($cmH_2O$), arterial base excess (mEq/L), noninvasive shock index (bpm/mmHg), partial thromboplastin (sec), arterial pH, total protein (g/dL), urine output (cc/kg/hr), hemoglobin amount (g/dL), noninvasive systolic blood pressure (mmHg), oxygen saturation index, height (cm), lactic acid amount (mg/dL), heart rate (bpm), noninvasive mean blood pressure (mmHg), invasive diastolic blood pressure (mmHg), $FiO_2$ Set percentage (%), daily weight (kg), invasive mean blood pressure (mmHg), invasive systolic blood pressure (mmHg), noninvasive diastolic blood pressure (mmHg), and/or other features. Feature component 24 is configured to obtain the one or more feature values based on information entered and/or selected via user interface 40, information stored in electronic storage and/or external resources 70 (e.g., the external clinical databases described above), the output signals of sensors 50 (described below), and/or other information. In some embodiments, feature component 24 is configured to obtain feature values in substantially real time as the feature values are being generated (e.g., by sensors included in system 10 and/or in external systems), as feature values become available to be obtained by feature component 24 (e.g., as feature values are stored in a database and/or entered via user interface 40), and/or at other times.

In some embodiments, obtaining feature values includes obtaining feature values directly. For example, a heart rate value may be stored in electronic storage 60 and directly obtained by feature component 24. As another example, feature component 24 may obtain a heart rate value directly from the output signals of a heart rate sensor (e.g., sensor 50). As a third example, feature component may directly obtain a heart rate value entered by a caregiver via user interface 40. In some embodiments, feature values are obtained indirectly. Indirectly obtaining feature values may include performing one or more calculations to determine feature values, and/or other methods for indirectly obtaining feature values. For example, an oxygen saturation index may be determined based on a combination of $SpO_2$ and $FiO_2$ values. As another example, noninvasive and invasive shock indices (nSI, iSI) may be derived from heart rate and noninvasive or invasive systolic blood pressure (nSBP, iSBP).

In some embodiments, obtaining feature values includes determining plausibility of values and/or other information and/or data obtained for the individual features. Determining plausibility includes filtering obtained values and/or other information based on predetermined value and/or information criteria, and retaining only the values and/or information that meets the predetermined criteria. This may reduce and and/or eliminate the inclusion of inaccurate information and/or values that do not reflect the physiological condition of subject 12. For example, feature component 24 may be configured to filter heart rate data to exclude any obtained heart rate values outside physiological ranges of 20-300 (these ranges are only examples and is not intended to be limiting) beats per minute (bpm). If feature component 24 obtains a heart rate value of 5 bpm, for example because there was an error in the heart rate measurement process, the 5 bpm heart rate is excluded by feature component 24. In some embodiments, the filtering is based on normal physiological ranges for the different features (e.g., the 20-300bpm range for heart rate).

In some embodiments, obtaining feature values includes learning which clinical features (e.g., the features described above) are relevant to and/or discriminative of hemodynamic instability in subject 12. In some embodiments, feature component 24 is configured to analyze a group of possible features using a training dataset and one or more machine learning algorithms (e.g., AdaBoost-abstain, linear discriminant analysis (LDA), and logistic/linear regression) to determine which features are relevant to and/or discriminative of hemodynamic instability. The training dataset may be clinical data for pediatric patients representative of subject 12. For example, system 10 was trained and tested using information from about 10,000 patients admitted to the pediatric intensive care unit, whose ages were between about 1 month and about 19 years old. During the training, system 10 received as inputs 61 clinical features from which it selected the most discriminative features (e.g., the 21 features described below). In addition, system 10 learned age-dependent thresholds (described below) for the individual clinical features. In some embodiments, feature component 24 is configured to determine that the relevant and/or discriminative features for hemodynamic instability in subject 12 include invasive shock index, mean airway pressure, arterial base excess, noninvasive shock index, partial thromboplastin, arterial pH, total protein, urine output, hemoglobin amount, noninvasive systolic blood pressure, oxygen saturation index, height, lactic acid amount, heart rate, noninvasive mean blood pressure, invasive diastolic blood pressure, $FiO_2$ Set percentage, daily weight, invasive mean blood pressure, invasive systolic blood pressure, noninvasive diastolic blood pressure, and/or other features.

Threshold component 26 is configured to determine one or more feature value thresholds for individual features that indicate risk of hemodynamic instability in subject 12. The determined feature value thresholds depend on the age of subject 12. The one or more feature value thresholds for individual features including heart rate, blood pressure, and shock index are quadratic functions of the age of subject 12. Other feature value thresholds for other features may be linear functions of the age of subject 12. For example, if a feature is heart rate, blood pressure, or shock index, the one or more feature value thresholds for an individual one of heart rate, blood pressure, or shock index are quadratic functions of the age of subject 12 determined using Equation 1.

$$\beta_1 + \beta_2\text{age} + \beta_3\text{age}^2 \qquad [1]$$

If the feature is one of the other features described above and/or other features, the one or more feature value thresholds for an individual feature are linear functions of age determined using Equation 2.

$$\beta_1 + \beta_2\text{age} \qquad [2]$$

During training (e.g., as described above), feature component 24 and/or threshold component 26 are configured to learn the parameters $\beta_1$, $\beta_2$, and $\beta_3$ by fitting a linear and/or quadratic function to the thresholds found for the individual clinical features for various age groups. In some embodiments, the thresholds determined by threshold component 26 are and/or are used to provide a final risk score of hemodynamic instability (described below). During training, system 10 may select a clinical feature more than once. Every time a feature is selected, system 10 has learned an age-dependent threshold (7), a bias (b), and a scaling factor ($\alpha$) for that selected feature. These parameters are then used in a bivariate classifier to determine the risk score of hemodynamic instability for that specific feature (described below).

Contribution component 28 is configured to determine feature contribution prediction scores for individual features. Individual feature contribution scores are aggregated (e.g., as described below) to determine the hemodynamic instability risk score. The feature contribution prediction scores are determined for the individual features based on whether the obtained feature values breach one or more of the determined feature value thresholds for the individual features. Contribution component 28 is configured such that the age of subject 12 and a given feature value are inputs for a bivariate classifier configured to output a corresponding feature contribution prediction score for the given feature. An individual bivariate classifier is composed of one or more decision stumps. In some embodiments, a decision stump *u* at threshold T denoted $u(x-T)$ is given by Equation 3, where x is an obtained feature value and T is a corresponding feature value threshold determined by threshold component 26 as described above.

$$u(x - \tau) = \begin{cases} 1, & x \geq \tau \\ -1, & x < \tau \end{cases} \qquad [3]$$

Using Equation 3, a positive value (e.g., 1, when a feature value breaches its corresponding threshold) increases the feature contribution score for that feature and is indicative of (e.g., "votes" for) hemodynamic instability. A negative value (e.g., -1, when the feature value does not breach its corresponding threshold) decreases the feature contribution score and is indicative of (e.g., "votes for") stability. With Equation 3 as described above, the individual bivariate classifiers for the individual features are of the form described by Equation 4.

$$f(x) = b + \alpha_1 u(x - \tau_1) + \alpha_2 u(x - \tau_2) + \ldots \qquad [4]$$

In Equation 4, b is a bias (constant value), $\alpha_i$ are scaling factors, and $\tau_i$ are age-dependent decision stump thresholds for an individual feature. As noted above, the thresholds $\tau_i$ may be learned using a machine learning algorithm and training data set. Similarly, the scaling factors $\alpha_i$ and bias b may also be learned using machine learning algorithms (which may be the same as that used to learn the threshold equations) and the clinical training data set (or another data set).

By way of a non-limiting example, Table I and Equations 5-9 show an example of using the bias (b), scaling factors ($\alpha$), and the parameters ($\beta_1$, $\beta_2$, $\beta_3$) to determine the age-dependent decision stump thresholds ($\tau$) and the feature contribution prediction scores $f(x)$ for noninvasive shock index (nSI) and pH (the bivariate classifier for nSI has two decision stumps with two corresponding alpha values and two corresponding thresholds, and the bivariate classifier for pH has one decision stump).

**Table I**

| Feature | b | $\beta_1$ | $\beta_2$ | $\beta_3$ | $\alpha$ |
|---------|-----|-----|-----|-----|-----|
| nSI | 0.0117 | 0.0022 | -0.0616 | 1.5463 | 0.2432 |
| nSI | 0.0519 | 0.0049 | -0.1161 | 1.6565 | -0.0102 |
| pH | 0.0856 | 0.0023 | 7.3212 | - | -0.1071 |

For nSI:

$$\tau_1 = 0.0022 - 0.0616 \cdot \text{age} + 1.5463 \cdot \text{age}^2 \qquad [5]$$

$$\tau_2 = 0.0049 - 0.1161 \cdot \text{age} + 1.6565 \cdot \text{age}^2 \qquad [6]$$

$$f(\text{nSI}) = 0.0117 + 0.2432 \cdot u(x_{\text{nSI}} - \tau_1) - 0.0102 \cdot u(x_{\text{nSI}} - \tau_2) \qquad [7]$$

For pH:

$$\tau_1 = 0.0023 + 7.3212 \cdot \text{age} \qquad [8]$$

$$f(\text{pH}) = 0.0856 - 0.1071 \cdot u(x_{\text{pH}} - \tau_1) \qquad [9]$$

Score component 30 is configured to aggregate the feature contribution prediction scores $f(x)$ for the individual features (e.g., f(nSI) + f(pH) + ...) to determine the hemodynamic instability risk score. The aggregation may comprise a summation of the feature contribution scores and/or other aggregations utilizing other mathematical operations performed using the feature contribution prediction scores. In some embodiments, the hemodynamic instability risk score for the subject is a single value from 0 to 1, wherein a value of 1 indicates relative hemodynamic instability and 0 indicates relative hemodynamic stability. In some embodiments, the hemodynamic instability risk score is a percentage on a 0-100 percentage point scale, wherein a value of 100% indicates relative hemodynamic instability and 0% indicates relative hemodynamic stability. These score formats are not intended to be limiting. The present application contemplates any score format configured to communicate relative hemodynamic stability and/or instability.

In some embodiments, score component 30 is configured to cause display of the hemodynamic instability risk score to a caregiver (e.g., doctor, nurse, parent, etc.) and/or other people associated with subject 12. In some embodiments, score component 30 is configured to cause display of the hemodynamic instability risk score over time for subject 12. In some embodiments, score component 30 is configured to overlay the hemodynamic instability risk score on a color coded background (e.g., red, yellow green) to indicate a severity of the hemodynamic instability risk based on the determined score and/or display the hemodynamic instability risk score in other ways. In some embodiments, score component 30 is configured to cause display of visual representations of how the one or more feature value thresholds change based on the age of subject 12. Score component 30 may cause display of the score, the visual representations, and/or other information via user interface 40, via a computing device associated with a caregiver, via a computing device associated with a care facility, and/or other devices.

In some embodiments, system 10 is configured to output a valid risk score of hemodynamic instability even if a clinical feature is missing (e.g., maybe that feature value was not measured by the nurse). If there is a missing feature value, system 10 does not take the contribution (e.g., $f(x,age)$) of that feature to produce a final score.

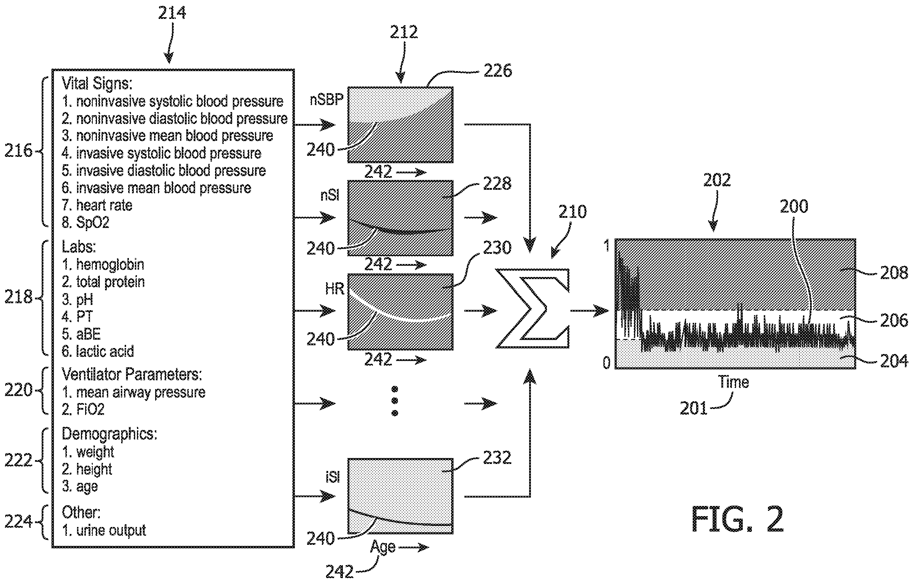

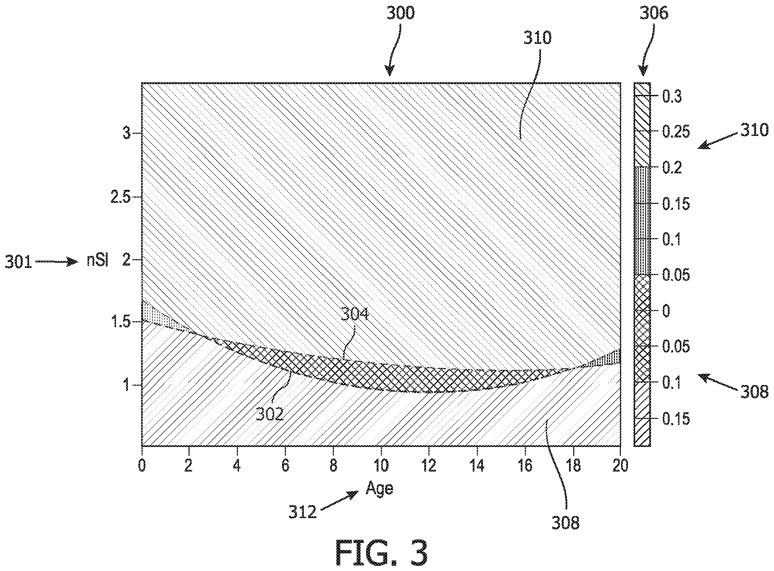

By way of a non-limiting example, FIG. 2 illustrates determining a hemodynamic instability risk score 200. Score 200 is displayed over time 201 on a color coded background 202 which indicates severity 204 (least severe), 206, 208 (most severe) of hemodynamic instability risk. In FIG. 2, score 200 is a summation 210 of feature contribution prediction scores 212 determined for individual features 214. FIG. 2 illustrates features 214 by category 216 (vital signs), 218 (lab values), 220 (ventilator parameters), 222 (demographics), 224 (other). FIG. 2 also visually illustrates bivariate classifiers including decision stumps for selected features noninvasive systolic blood pressure (nSBP) 226, noninvasive shock index (nSI) 228, heart rate (HR) 230, and invasive shock index (iSI) 232. Illustrations 226, 228, 230, and 232 show 240 how the thresholds for the individual features vary with the age 242 of the subject. This is further illustrated in FIG. 3

FIG. 3 is a visual illustration 300 of a bivariate classifier including two decision stumps (e.g., nSI was selected twice) for noninvasive shock index 301 (nSI). As described above, individual decision stumps are defined by an age dependent threshold 302, 304 (one threshold per decision stump), a bias (not shown in FIG. 3), and a scaling factor (not shown in FIG. 3). FIG. 3 also illustrates possible feature contribution prediction scores 306 for nSI (depending on the nSI value and the age of the subject) and is color coded 308, 310 to indicate the relative risk (e.g., the area noted by 308 is low risk and the area noted by 310 is high risk) of hemodynamic instability for different nSI values 301 for subjects of different ages 312.

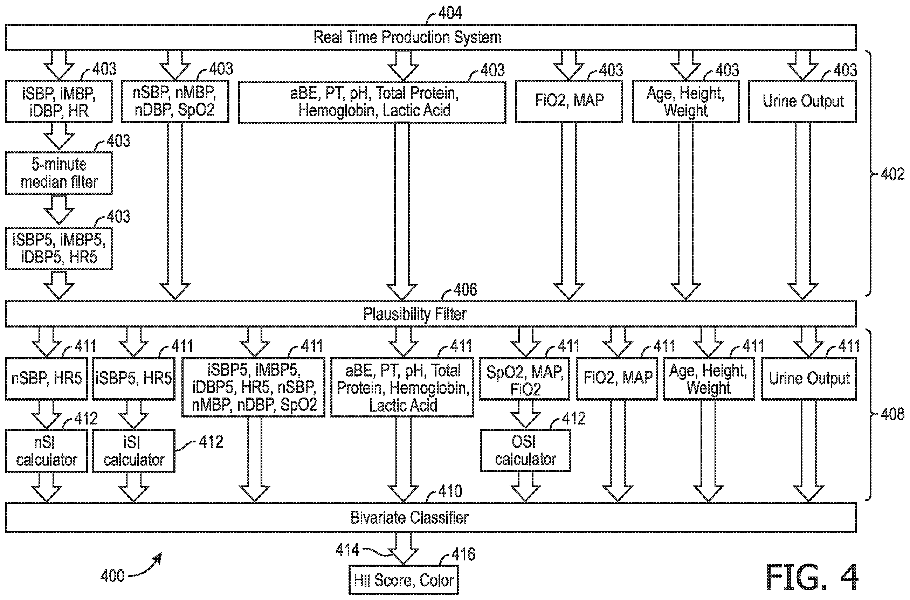

FIG. 4 illustrates a summary of the operations 400 performed by system 10 (shown in FIG. 1). As shown in FIG. 4, system 10 obtains 402 feature values 403 from one or more systems 404 (e.g., system 10 and/or external systems as described above) generating the feature values and/or information related to the feature values. System 10 determines 406 whether the obtained feature values are plausible and then uses 408 the plausible features 411 and the age of the subject as inputs for bivariate classifiers 410 for the individual features. In some embodiments, after determining that the obtained feature values are plausible, system 10 determines one or more feature values 412 based on previously obtained feature values and/or information related to the feature values. Feature contribution prediction scores output by bivariate classifiers 410 for the individual features are aggregated 414 to determine the hemodynamic instability risk score (HII score) 416, which may be displayed on a color coded risk scale (e.g., as described above) and/or by other methods.

Returning to FIG. 1, user interface 40 is configured to receive information from and/or provide information to one or more users (e.g., subject 12, caregivers, etc.) of system 10. User interface 40 is configured to provide an interface between system 10 and caregivers, subject 12, and/or other users through which caregivers, subject 12, and/or other users may provide information to and receive information from system 10. This enables data, cues, results, and/or instructions and any other communicable items, collectively referred to as "information," to be communicated between a user (e.g., a caregiver, subject 12, and/or other users) and processor 20, and/or other components of system 10. For example, the hemodynamic instability risk score may be communicated to caregiver, subject 12, and/or other users via user interface 40.

Examples of interface devices suitable for inclusion in user interface 40 comprise a graphical user interface, a display, a touchscreen, a keypad, buttons, switches, a keyboard, knobs, levers, speakers, a microphone, an indicator light, an audible alarm, a printer, a haptic feedback device, and/or other interface devices. In some embodiments, user interface 40 comprises a plurality of separate interfaces. For example, user interface 40 may comprise a plurality of different interfaces associated with a plurality of computing devices associated with different caregivers; an interface that is part of a computing device associated with a care facility; an interface associated with processor 20, electronic storage 60, external resources 70, sensors 50, and/or other components of system 10; an interface included in a server that also includes processor 20 and/or electronic storage 60; and/or other interfaces. User interface 40 is configured such that a plurality of caregivers, subject 12, and/or other users may provide information to and receive information from system 10 via the individual ones of the plurality of user interfaces. In some embodiments, user interface 40 comprises at least one interface that is provided integrally with processor 20 and/or other components of system 10.

It is to be understood that other communication techniques, either hardwired or wireless, are also contemplated by the present disclosure as user interface 40. For example, the present disclosure contemplates that user interface 40 may be integrated with a removable storage interface provided by electronic storage 60. In this example, information may be loaded into system 10 from removable storage (e.g., a smart card, a flash drive, a removable disk, etc.) that enables the user(s) to customize the implementation of system 10. Other exemplary input devices and techniques adapted for use with system 10 as user interface 40 comprise, but are not limited to, an RS-232 port, RF link, an IR link, modem (telephone, cable or other). In short, any technique for communicating information with system 10 is contemplated by the present disclosure as user interface 40.

Sensor(s) 50 are configured to generate output signals conveying information related to one or more physiological characteristics of subject 12, and/or other information indicative of the health status of subject 12. In some embodiments, the output signals are received by processor 20, stored in electronic storage 60, received and stored by one or more servers included in external resources 70, and/or communicated to other devices. The physiological characteristics of subject 12 include vital signs and/or physiological parameters including and/or related to weight of subject 12, blood pressure of subject 12, heart rate of subject 12, respiration rate of subject 12, blood chemistry of subject 12, hydration of subject 12, a respiration rate/output of subject 12, a blood oxygen level of subject 12, skin conductance and/or skin temperature of subject 12, body temperature of subject 12, the joint/muscular flexibility of subject 12, the blood circulation of subject 12, the cardiac output of subject 12, the relative health and/or sickness of subject 12, brain activity of subject 12, cardiovascular activity of subject 12, and/or other parameters. Sensors 50 may comprise one or more sensors that measure such parameters directly. For example, sensors 50 may be and/or include a heart rate sensor located on the chest of subject 12. Sensors 50 may comprise one or more sensors that generate output signals conveying information related to physiological characteristics of subject 12 indirectly. For example, one or more sensors 50 may generate an output with vital signs information based on movement of subject 12 (e.g. movement detected via actigraphy signals from a bracelet on a wrist of subject 12 may indicate a higher heart rate). In some embodiments, sensors 50 may be and/or include one or more of a heart rate monitor, a blood pressure monitor, a weight scale, blood glucose meter, oxygen saturation measurement, and/or other sensors.

Although sensors 50 are illustrated at a single location near subject 12, this is not intended to be limiting. Sensors 50 may include sensors disposed in a plurality of locations, such as for example, coupled (in a removable manner) with clothing of subject 12, worn by subject 12 (e.g., as a headband, wristband, etc.), positioned to point at subject 12 (e.g., a camera that conveys output signals related to heart rate of subject 12), and/or in other locations. In some embodiments, sensors 50 are associated with a hospital and/or other care facility (e.g., a doctor's office), a caregiver (e.g., sensors 14 may be included in equipment used by a doctor, nurse, a caregiving family member, etc.), medical transportation services (e.g., sensors 14 may be included in an ambulance), subject 12 (e.g., sensors in a smartphone associated with subject 12 and/or the parent of subject 12), and/or include other sensors. Sensors 50 may generate output signals continuously, at predetermined intervals, responsive to presence of and/or interaction with subject 12, and/or at other times. In some embodiments, system 10 may not include sensors 50.

Electronic storage 60 comprises electronic storage media that electronically stores information. The electronic storage media of electronic storage 60 may comprise one or both of system storage that is provided integrally (i.e., substantially non-removable) with system 10 and/or removable storage that is removably connectable to system 10 via, for example, a port (e.g., a USB port, a firewire port, etc.) or a drive (e.g., a disk drive, etc.). Electronic storage 60 may be (in whole or in part) a separate component within system 10, or electronic storage 60 may be provided (in whole or in part) integrally with one or more other components of system 10 (e.g., user interface 40, processor 20, etc.). In some embodiments, electronic storage may be located in a server together with processor 20, in a server that is part of external resources 70, in a computing device associated with one or more caregivers, subject 12, and/or other users, and/or in other locations. Electronic storage 60 may comprise one or more of optically readable storage media (e.g., optical disks, etc.), magnetically readable storage media (e.g., magnetic tape, magnetic hard drive, floppy drive, etc.), electrical charge-based storage media (e.g., EPROM, RAM, etc.), solid-state storage media (e.g., flash drive, etc.), and/or other electronically readable storage media. Electronic storage 60 may store software algorithms, information determined by processor 20, information received via user interface 40 and/or external computing systems, information received from external resources 70, information received from sensors 50, and/or other information that enables system 10 to function as described herein.

External resources 70 includes sources of information (e.g., databases, websites, etc.), external entities participating with system 10 (e.g., a medical records system of a health care provider), medical equipment configured to communicate with external systems, one or more servers outside of system 10, a network (e.g., the internet), electronic storage, equipment related to Wi-Fi technology, equipment related to Bluetooth® technology, data entry devices, sensors, scanners, computing devices associated with individual users, and/or other resources. For example, in some embodiments, external resources 70 include one or more external patient medical information databases that include the age of subject 12, feature values associated with subject 12, and/or other information. In some implementations, some or all of the functionality attributed herein to external resources 70 may be provided by resources included in system 10. External resources 70 may be configured to communicate with processors 20, user interface 40, sensors 50, electronic storage 60, and/or other components of system 10 via wired and/or wireless connections, via a network (e.g., a local area network and/or the internet), via cellular technology, via Wi-Fi technology, and/or via other resources.

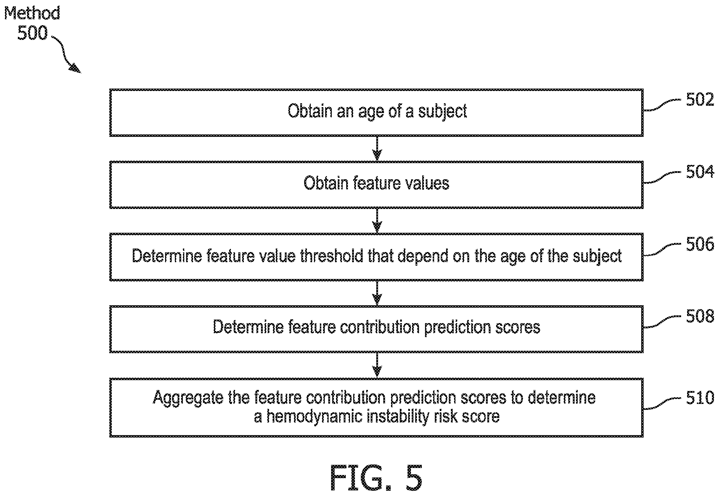

FIG. 5 illustrates a method 500 for determining a hemodynamic instability risk score for a pediatric patient with a determination system. The system comprises one or more hardware processors and/or other components. The one or more hardware processors are configured by machine readable instructions to execute computer program components. The computer program components comprise an age component, a feature component, a threshold component, a contribution component, a score component, and/or other components. The operations of method 500 presented below are intended to be illustrative. In some embodiments, method 500 may be accomplished with one or more additional operations not described, and/or without one or more of the operations discussed. Additionally, the order in which the operations of method 500 are illustrated in FIG. 5 and described below is not intended to be limiting.

In some embodiments, method 500 may be implemented in one or more processing devices (e.g., a digital processor, an analog processor, a digital circuit designed to process information, an analog circuit designed to process information, a state machine, and/or other mechanisms for electronically processing information). The one or more processing devices may include one or more devices executing some or all of the operations of method 500 in response to instructions stored electronically on an electronic storage medium. The one or more processing devices may include one or more devices configured through hardware, firmware, and/or software to be specifically designed for execution of one or more of the operations of method 500.

At an operation 502, an age of the subject is obtained. In some embodiments, operation 502 is performed by a processor component the same as or similar to age component 22 (shown in FIG. 1 and described herein).

At an operation 504, feature values are obtained. Obtaining feature values may include obtaining feature values for one or more features associated with physiological characteristics of the subject. The one or more features include invasive shock index, mean airway pressure, arterial base excess, noninvasive shock index, partial thromboplastin, arterial pH, total protein, urine output, hemoglobin amount, noninvasive systolic blood pressure, oxygen saturation index, height, lactic acid amount, heart rate, noninvasive mean blood pressure, invasive diastolic blood pressure, $FiO_2$ Set percentage, daily weight, invasive mean blood pressure, invasive systolic blood pressure, noninvasive diastolic blood pressure, and/or other features. In some embodiments, responsive to not obtaining a given feature value, the system is configured to exclude the given feature when (as described below) determining the one or more feature value thresholds for individual features, determining the feature contribution prediction scores for the individual features, and aggregating the feature contribution prediction scores (for the features that were available) to determine the hemodynamic instability risk score for the subject. In some embodiments, operation 504 includes generating, with one or more sensors, output signals conveying information related to the one or more features associated with physiological characteristics of the subject. In such embodiments, the one or more feature values may at least in part be obtained via the output signals. In some embodiments, operation 504 is performed by a processor component the same as or similar to feature component 24 (shown in FIG. 1 and described herein).

At an operation 506, feature value thresholds that depend on the age of the subject are determined. Operation 506 includes determining one or more feature value thresholds for individual features that indicate risk of hemodynamic instability in the subject. The one or more feature value thresholds for individual features including heart rate, blood pressure, and shock index are quadratic functions of the age of the subject. Other feature value thresholds for other features may be linear functions of the age of the subject. In some embodiments, operation 506 is performed by a processor component the same as or similar to threshold component 26 (shown in FIG. 1 and described herein).

At an operation 508, feature contribution prediction scores are determined. The feature contribution prediction scores are determined for the individual features based on whether the obtained feature values breach one or more of the determined feature value thresholds for the individual features. The age of the subject and a given feature value are inputs for a bivariate classifier configured to output a corresponding feature contribution prediction score for the given feature. In some embodiments, operation 508 is performed by a processor component the same as or similar to contribution component 28 (shown in FIG. 1 and described herein).

At an operation 510, the feature contribution scores are aggregated to determine the hemodynamic instability risk score. In some embodiments, the hemodynamic instability risk score for the subject is a single value from 0 to 1, wherein a value of 1 indicates relative hemodynamic instability and 0 indicates relative hemodynamic stability. In some embodiments, the hemodynamic instability risk score is a percentage on a 0-100 percentage point scale, wherein a value of 100% indicates relative hemodynamic instability and 0% indicates relative hemodynamic stability. These score formats are not intended to be limiting. The present application contemplates any score format configured to communicate relative hemodynamic stability and/or instability. In some embodiments, operation 510 includes displaying (and/or causing display of) the hemodynamic instability risk score to a caregiver associated with the subject, and displaying visual representations of how the one or more feature value thresholds change based on the age of the subject with a user interface. In some embodiments, operation 510 is performed by a processor component the same as or similar to score component 30 (shown in FIG. 1 and described herein).

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

Although the description provided above provides detail for the purpose of illustration based on what is currently considered to be the most practical and preferred embodiments, it is to be understood that such detail is solely for that purpose and that the disclosure is not limited to the expressly disclosed embodiments, but, on the contrary, is intended to cover modifications and equivalent arrangements that are within the scope of the appended claims. For example, it is to be understood that the present disclosure contemplates that, to the extent possible, one or more features of any embodiment can be combined with one or more features of any other embodiment.

**Claims**

1. A system (10) configured to determine a hemodynamic instability risk score for a pediatric subject (12), the system comprising:
   one or more hardware processors (20) configured by machine-readable instructions to:

   obtain an age of the subject;
   obtain feature values for a plurality of features associated with physiological characteristics of the subject;
   input the age of the subject and each feature value into a respective bivariate classifier that is configured to output a respective feature contribution prediction score for each feature, wherein each bivariate classifier determines one or more feature value thresholds for the respective feature that indicates risk of hemodynamic instability in the subject, wherein the feature value thresholds are determined based on the age of the subject, such that the one or more feature value thresholds for individual features including heart rate, blood pressure, and shock index are quadratic functions of the age of the subject;
   use each bivariate classifier to determine a feature contribution prediction score for the respective feature based on whether the obtained feature value breaches the one or more of the determined feature value thresholds for the individual features; and
   aggregate the feature contribution prediction scores to determine the hemodynamic instability risk score for the subject.

2. The system of claim 1, wherein the one or more hardware processors are further configured such that the plurality of features include one or more of invasive shock index, mean airway pressure, arterial base excess, noninvasive shock index, partial thromboplastin, arterial pH, total protein, urine output, hemoglobin amount, noninvasive systolic blood pressure, oxygen saturation index, height, lactic acid amount, heart rate, noninvasive mean blood pressure, invasive diastolic blood pressure, $FiO_2$ Set percentage, daily weight, invasive mean blood pressure, invasive systolic blood pressure, or noninvasive diastolic blood pressure.

3. The system of claim 1, wherein the one or more hardware processors are configured such that the hemodynamic instability risk score for the subject is a single value from 0 to 1, wherein a value of 1 indicates relative hemodynamic instability and 0 indicates relative hemodynamic stability.

4. The system of claim 1, wherein the one or more hardware processors are further configured to, responsive to not obtaining a given feature value in the plurality of obtained feature values, exclude the given feature when determining the one or more feature value thresholds for individual features, determining the feature contribution prediction scores for the individual features, and aggregating the feature contribution prediction scores to determine the hemodynamic instability risk score for the subject.

5. The system of claim 1, further comprising:

a user interface (40) configured to display the hemodynamic instability risk score to a caregiver associated with the subject, and visual representations of how the one or more feature value thresholds change based on the age of the subject; and

one or more sensors (50) configured to generate output signals conveying information related to the one or more features associated with physiological characteristics of the subject, wherein the one or more hardware processors are configured to obtain the one or more feature values via the output signals.

6. A method for determining a hemodynamic instability risk score for a pediatric subject (12) with a determination system (10), the system comprising one or more hardware processors (20) configured by machine-readable instructions to execute the method comprising:

obtaining an age of the subject;

obtaining feature values for a plurality of features associated with physiological characteristics of the subject;

inputting the age of the subject and each feature value into a respective bivariate classifier that outputs a respective feature contribution prediction score for each feature, wherein each bivariate classifier determines one or more feature value thresholds for the respective feature that indicates risk of hemodynamic instability in the subject, wherein the feature value thresholds are determined based on the age of the subject, wherein the one or more feature value thresholds for individual features including heart rate, blood pressure, and shock index are quadratic functions of the age of the subject;

using each bivariate classifier to determine a feature contribution prediction score for the respective feature based on whether the obtained feature value breaches the one or more of the determined feature value thresholds for the individual features; and

aggregating the feature contribution prediction scores to determine the hemodynamic instability risk score for the subject.

7. The method of claim 6, wherein the plurality of features include one or more of invasive shock index, mean airway pressure, arterial base excess, noninvasive shock index, partial thromboplastin, arterial pH, total protein, urine output, hemoglobin amount, noninvasive systolic blood pressure, oxygen saturation index, height, lactic acid amount, heart rate, noninvasive mean blood pressure, invasive diastolic blood pressure, $FiO_2$ Set percentage, daily weight, invasive mean blood pressure, invasive systolic blood pressure, or noninvasive diastolic blood pressure.

8. The method of claim 6, wherein the hemodynamic instability risk score for the subject is a single value from 0 to 1, wherein a value of 1 indicates relative hemodynamic instability and 0 indicates relative hemodynamic stability.

9. The method of claim 6, further comprising, responsive to not obtaining a given feature value in the plurality of obtained feature values, excluding the given feature when determining the one or more feature value thresholds for individual features, determining the feature contribution prediction scores for the individual features, and aggregating the feature contribution prediction scores to determine the hemodynamic instability risk score for the subject.

10. The method of claim 6, further comprising:

displaying the hemodynamic instability risk score to a caregiver associated with the subject, and displaying visual representations of how the one or more feature value thresholds change based on the age of the subject with a user interface (40); and

generating, with one or more sensors (50), output signals conveying information related to the one or more features associated with physiological characteristics of the subject, wherein the one or more feature values are obtained via the output signals.

**Patentansprüche**

1. System (10), das konfiguriert ist, um eine hämodynamische Instabilitätsrisikobewertung für ein pädiatrisches Subjekt (12) zu bestimmen, wobei das System umfasst:

ein oder mehrere Hardwareprozessoren (20), die durch maschinenlesbare Anweisungen konfiguriert sind, um:

Erhalten eines Alters des Subjekts;

Erhalten von Merkmalswerten für eine Vielzahl von Merkmalen, die mit physiologischen Eigenschaften des Subjekts verbunden sind;

Eingeben des Alters des Subjekts und jedes Merkmalswertes in einen jeweiligen bivariaten Klassifikator, der konfiguriert ist, um für jedes Merkmal eine jeweilige Merkmalsbeitragsvorhersagebewertung auszugeben, wobei jeder bivariate Klassifikator eine oder mehrere Merkmalswertschwellen für das jeweilige Merkmal bestimmt, die das Risiko einer hämodynamischen Instabilität in dem Subjekt anzeigen, wobei die Merkmalswertschwellen basierend auf dem Alter des Subjekts bestimmt werden, so dass die eine oder die mehreren Merkmalswertschwellen für einzelne Merkmale, einschließlich Herzfrequenz, Blutdruck und Schockindex, quadratische Funktionen des Alters des Subjekts sind;

Verwenden jedes bivariaten Klassifikators, um eine Merkmalsbeitragsvorhersagebewertung für das jeweilige Merkmal zu bestimmen, basierend darauf, ob der erhaltene Merkmalswert eine oder mehrere der ermittelten Merkmalswertschwellen für die einzelnen Merkmale verletzt; und

Aggregieren der Merkmalsbeitragsvorhersagebewertungen, um die hämodynamische Instabilitätsrisikobewertung für das Subjekt zu bestimmen.

2. System nach Anspruch 1, wobei der eine oder die mehreren Hardwareprozessoren ferner so konfiguriert sind, dass die Vielzahl von Merkmalen umfasst einen oder mehrere des invasiven Schockindex, des mittleren Atemwegsdrucks, des arteriellen Basenüberschusses, des nichtinvasiven Schockindex, des partiellen Thromboplastins, des arteriellen pH-Werts, des Gesamtproteins, des Urinausstoßes, der Hämoglobinmenge, des nichtinvasiven systolischen Blutdrucks, des Sauerstoffsättigungsindex, der Körpergröße, der Milchsäuremenge, der Herzfrequenz, des nichtinvasiven mittleren Blutdrucks, des invasiven diastolischen Blutdrucks, des festgelegten $FiO_2$-Prozentsatzes, des Tagesgewichts, des invasiven mittleren Blutdrucks, des invasiven systolischen Blutdrucks oder des nichtinvasiven diastolischen Blutdrucks.

3. System nach Anspruch 1, wobei der eine oder die mehreren Hardwareprozessoren so konfiguriert sind, dass die hämodynamische Instabilitätsrisikobewertung für das Subjekt ein Einzelwert von 0 bis 1 ist, wobei ein Wert von 1 eine relative hämodynamische Instabilität anzeigt und 0 eine relative hämodynamische Stabilität anzeigt.

4. System nach Anspruch 1, wobei als Reaktion auf das Nichterhalten eines gegebenen Merkmalswerts in der Vielzahl von erhaltenen Merkmalswerten, der eine oder die mehreren Hardwareprozessoren ferner konfiguriert sind, zum Ausschließen des gegebenen Merkmals beim Bestimmen der einen oder mehreren Merkmalswertschwellen für einzelne Merkmale, Bestimmen der Merkmalsbeitragsvorhersagebewertungen für die einzelnen Merkmale, und Aggregieren der Merkmalsbeitragsvorhersagebewertungen, um die hämodynamische Instabilitätsrisikobewertung für das Subjekt zu bestimmen.

5. System nach Anspruch 1, ferner umfassend:

eine Benutzerschnittstelle (40), die konfiguriert ist, um einer mit dem Subjekt verbundenen Pflegekraft die hämodynamische Instabilitätsrisikobewertung anzuzeigen, und visuelle Darstellungen, wie sich die eine oder die mehreren Merkmalswertschwellen basierend auf dem Alter des Subjekts ändern; und

einen oder mehrere Sensoren (50), die konfiguriert sind, um Ausgangssignale zu erzeugen, die Informationen über das eine oder die mehreren Merkmale übertragen, die mit physiologischen Eigenschaften des Subjekts verbunden sind, wobei der eine oder die mehreren Hardwareprozessoren konfiguriert sind, um den einen oder die mehreren Merkmalswerte über die Ausgangssignale zu erhalten.

6. Verfahren zum Bestimmen einer hämodynamischen Instabilitätsrisikobewertung für ein pädiatrisches Subjekt (12) mit einem Bestimmungssystem (10), wobei das System einen oder mehrere Hardwareprozessoren (20) umfasst, die durch maschinenlesbare Anweisungen konfiguriert sind, um das Verfahren auszuführen, umfassend:

Erhalten eines Alters des Subjekts;

Erhalten von Merkmalswerten für eine Vielzahl von Merkmalen, die mit physiologischen Eigenschaften des Subjekts verbunden sind;

Eingabe des Alters des Subjekts und jedes Merkmalswerts in einen jeweiligen bivariaten Klassifikator, der eine jeweilige Merkmalsbeitragsvorhersagebewertung für jedes Merkmal ausgibt, wobei jeder bivariate Klassifikator eine oder mehrere Merkmalswertschwellen für das jeweilige Merkmal bestimmt, die das Risiko einer hämodynamischen Instabilität in dem Subjekt anzeigen, wobei die Merkmalswertschwellen basierend auf dem Alter des Subjekts bestimmt werden, wobei die eine oder die mehreren Merkmalswertschwellen für einzelne Merkmale, einschließlich Herzfrequenz, Blutdruck und Schockindex, quadratische Funktionen des Alters des Subjekts sind;

Verwenden jedes bivariaten Klassifikators zum Bestimmen einer Merkmalsbeitragsvorhersagebewertung für das jeweilige Merkmal auf der Grundlage, ob der erhaltene Merkmalswert einen oder mehrere der ermittelten Merkmalswertschwellen für die einzelnen Merkmale verletzt; und

Aggregieren der Merkmalsbeitragsvorhersagebewertungen, um die hämodynamische Instabilitätsrisikobewertung für das Subjekt zu bestimmen.

**7.** Verfahren nach Anspruch 6, wobei die Vielzahl von Merkmalen umfasst einen oder mehrere des invasiven Schockindex, des mittleren Atemwegsdrucks, des arteriellen Basenüberschusses, des nichtinvasiven Schockindex, des partiellen Thromboplastins, des arteriellen pH-Werts, des Gesamtproteins, des Urinausstoßes, der Hämoglobinmenge, des nichtinvasiven systolischen Blutdrucks, des Sauerstoffsättigungsindex, der Körpergröße, der Milchsäuremenge, der Herzfrequenz, des nichtinvasiven mittleren Blutdrucks, des invasiven diastolischen Blutdrucks, des festgelegten $FiO_2$-Prozentsatzes, des Tagesgewichts, des invasiven mittleren Blutdrucks, des invasiven systolischen Blutdrucks oder des nichtinvasiven diastolischen Blutdrucks.

**8.** Verfahren nach Anspruch 6, wobei die hämodynamische Instabilitätsrisikobewertung für das Subjekt ein Einzelwert von 0 bis 1 ist, wobei ein Wert von 1 eine relative hämodynamische Instabilität anzeigt und 0 eine relative hämodynamische Stabilität anzeigt.

**9.** Verfahren nach Anspruch 6, ferner umfassend, als Reaktion auf das Nichterhalten eines gegebenen Merkmalswerts in der Vielzahl von erhaltenen Merkmalswerten, Ausschließen des gegebenen Merkmals beim Bestimmen der einen oder mehreren Merkmalswertschwellen für einzelne Merkmale, Bestimmen der Merkmalsbeitragsvorhersagebewertungen für die einzelnen Merkmale und Aggregieren der Merkmalsbeitragsvorhersagebewertungen, um die hämodynamische Instabilitätsrisikobewertung für das Subjekt zu bestimmen.

**10.** Verfahren nach Anspruch 6, ferner umfassend:

Anzeigen der hämodynamische Instabilitätsrisikobewertung für eine mit dem Subjekt verbundene Pflegekraft und Anzeigen visueller Darstellungen, wie sich die Schwellenwerte für einen oder mehrere Merkmalswerte basierend auf dem Alter des Subjekts mit einer Benutzeroberfläche ändern (40); und

Erzeugen von Ausgangssignalen mit einem oder mehreren Sensoren (50), die Informationen über das eine oder die mehreren Merkmale vermitteln, die mit physiologischen Eigenschaften des Subjekts verbunden sind, wobei der eine oder die mehreren Merkmalswerte über die Ausgangssignale erhalten werden.

**Revendications**

**1.** Système (10) configuré pour déterminer un score de risque d'instabilité hémodynamique pour un sujet pédiatrique (12), le système comprenant:

un ou plusieurs processeurs matériels (20) configurés par des instructions lisibles par machine pour:

obtenir un âge du sujet;

obtenir des valeurs de caractéristique pour une pluralité de caractéristiques associées à des caractéristiques physiologiques du sujet;

entrer l'âge du sujet et chaque valeur de caractéristique dans un classificateur bivarié respectif qui est configuré pour sortir un score de prédiction de contribution de caractéristique respectif pour chaque caractéristique, où chaque classificateur bivarié détermine un ou plusieurs seuils de valeurs de caractéristique pour la caractéristique respective qui indique un risque d'instabilité hémodynamique chez le sujet, les seuils de valeurs de caractéristique étant déterminés sur la base de l'âge du sujet, de sorte que l'un ou les plusieurs seuils de valeurs de caractéristique pour des caractéristiques individuelles comprenant la fréquence cardiaque, la pression sanguine, et l'index de choc sont des fonctions quadratiques de l'âge du sujet matière;

utiliser chaque classificateur bivarié pour déterminer un score de prédiction de contribution de caractéristique pour la caractéristique respective sur la base du fait que la valeur de caractéristique obtenue dépasse l'un ou les plusieurs seuils de valeur de caractéristique déterminés pour les caractéristiques individuelles; et

agréger les scores de prédiction de contribution de caractéristiques pour déterminer le score de risque d'instabilité hémodynamique pour le sujet.

**2.** Système selon la revendication 1, dans lequel l'un ou les plusieurs processeurs matériels sont en outre configurés de telle sorte que la pluralité de caractéristiques comprend un ou plusieurs parmi l'index de choc invasif, la pression moyenne des voies aériennes, l'excès de base artérielle, l'index de choc non invasif, la thromboplastine partielle, le pH artériel, les protéines totales, le débit urinaire, la quantité d'hémoglobine, la pression sanguine systolique non invasive, l'indice de saturation en oxygène, la taille, la quantité d'acide lactique, la fréquence cardiaque, la pression sanguine moyenne non invasive, la pression sanguine diastolique invasive, le pourcentage établi en FiO$_2$, le poids quotidien, la pression sanguine moyenne invasive, la pression sanguine systolique invasive, ou la pression sanguine diastolique non invasive.

**3.** Système selon la revendication 1, dans lequel l'un ou les plusieurs processeurs matériels sont configurés de telle sorte que le score de risque d'instabilité hémodynamique pour le sujet est une valeur unique de 0 à 1, où une valeur de 1 indique une instabilité hémodynamique relative et 0 indique une stabilité hémodynamique relative.

**4.** Système selon la revendication 1, dans lequel l'un ou les plusieurs processeurs matériels sont en outre configurés pour, en réponse au fait de ne pas obtenir une valeur de caractéristique donnée parmi la pluralité de valeurs de caractéristique obtenues, exclure la caractéristique donnée lors de la détermination de l'un ou des plusieurs seuils de valeurs de caractéristique pour des caractéristiques individuelles, déterminer les scores de prédiction de contribution de caractéristique pour les caractéristiques individuelles, et agréger les scores de prédiction de contribution de caractéristique pour déterminer le score de risque d'instabilité hémodynamique pour le sujet.

**5.** Système selon la revendication 1, comprenant en outre:

une interface utilisateur (40) configurée pour afficher le score de risque d'instabilité hémodynamique à un soignant associé au sujet, et des représentations visuelles de la façon dont l'un ou les plusieurs seuils de valeur de caractéristique changent sur la base de l'âge du sujet; et
un ou plusieurs capteurs (50) configurés pour générer des signaux de sortie véhiculant des informations relatives à l'une ou aux plusieurs caractéristiques associées aux caractéristiques physiologiques du sujet, où l'un ou les plusieurs processeurs matériels sont configurés pour obtenir l'une ou les plusieurs valeurs de caractéristique via les signaux de sortie.

**6.** Procédé pour déterminer un score de risque d'instabilité hémodynamique pour un sujet pédiatrique (12) avec un système de détermination (10), le système comprenant un ou plusieurs processeurs matériels (20) configurés par des instructions lisibles par machine pour exécuter le procédé consistant à:

obtenir un âge du sujet;
obtenir des valeurs de caractéristique pour une pluralité de caractéristiques associées à des caractéristiques physiologiques du sujet;
entrer l'âge du sujet et chaque valeur de caractéristique dans un classificateur bivarié respectif qui sort un score de prédiction de contribution de caractéristique respectif pour chaque caractéristique, où chaque classificateur bivarié détermine un ou plusieurs seuils de valeur de caractéristique pour la caractéristique respective qui indique un risque d'instabilité hémodynamique chez le sujet, où les seuils de valeur de caractéristique sont déterminés sur la base de l'âge du sujet, où l'un ou les plusieurs seuils de valeurs de caractéristique pour des caractéristiques individuelles comprenant la fréquence cardiaque, la pression sanguine, et l'index de choc sont des fonctions quadratiques de l'âge du sujet;
utiliser chaque classificateur bivarié pour déterminer un score de prédiction de contribution de caractéristique pour la caractéristique respective sur la base du fait que la valeur de caractéristique obtenue dépasse l'un ou les plusieurs seuils de valeur de caractéristique déterminés pour les caractéristiques individuelles; et
agréger les scores de prédiction de contribution de caractéristique pour déterminer le score de risque d'instabilité hémodynamique pour le sujet.

**7.** Procédé selon la revendication 6, dans lequel la pluralité de caractéristiques comprend un ou plusieurs parmi l'index de choc invasif, la pression moyenne des voies aériennes, l'excès de base artérielle, l'index de choc non invasif, la thromboplastine, le pH artériel, les protéines totales, le débit urinaire, la quantité d'hémoglobine, la pression sanguine systolique non invasive, l'indice de saturation en oxygène, la taille, la quantité d'acide lactique, la fréquence cardiaque, la pression sanguine moyenne non invasive, la pression sanguine diastolique invasive, le pourcentage établi en FiO$_2$, le poids quotidien, la pression sanguine moyenne invasive, la pression sanguine systolique invasive, ou la pression sanguine diastolique non invasive.

8. Procédé selon la revendication 6, dans lequel le score de risque d'instabilité hémodynamique pour le sujet est une valeur unique de 0 à 1, où une valeur de 1 indique une instabilité hémodynamique relative et 0 indique une stabilité hémodynamique relative.

9. Procédé selon la revendication 6, comprenant en outre, en réponse au fait de ne pas obtenir une valeur de caractéristique donnée parmi la pluralité de valeurs de caractéristique obtenues, excluant la caractéristique donnée lors de la détermination de l'un ou des plusieurs seuils de valeur de caractéristique pour des caractéristiques individuelles, déterminer les scores de prédiction de contribution de caractéristique pour les caractéristiques individuelles, et agréger les scores de prédiction de contribution de caractéristique pour déterminer le score de risque d'instabilité hémodynamique pour le sujet.

10. Procédé selon la revendication 6, comprenant en outre:

afficher le score de risque d'instabilité hémodynamique à un soignant associé au sujet, et afficher des représentations visuelles de la façon dont l'un ou les plusieurs seuils de valeur caractéristique changent sur la base de l'âge du sujet avec une interface utilisateur (40); et
générer, avec un ou plusieurs capteurs (50), des signaux de sortie véhiculant des informations relatives à l'une ou aux plusieurs caractéristiques associées aux caractéristiques physiologiques du sujet, où l'une ou les plusieurs valeurs de caractéristique sont obtenues via les signaux de sortie.

FIG. 1

FIG. 2

FIG. 3

18

FIG. 4

Method
500

Obtain an age of a subject — 502

Obtain feature values — 504

Determine feature value threshold that depend on the age of the subject — 506

Determine feature contribution prediction scores — 508

Aggregate the feature contribution prediction scores to determine a hemodynamic instability risk score — 510

# FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2014323846 A
- US 2015145691 A